(19) Europäisches Patentamt
European Patent Office
Office européen des brevets

(11) **EP 3 246 040 B1**

(12) **EUROPEAN PATENT SPECIFICATION**

(45) Date of publication and mention of the grant of the patent:
**11.11.2020 Bulletin 2020/46**

(21) Application number: **15878114.6**

(22) Date of filing: **08.09.2015**

(51) Int Cl.:
**A61K 36/36** (2006.01)          **A61P 15/00** (2006.01)
**A61P 15/12** (2006.01)

(86) International application number:
**PCT/KR2015/009464**

(87) International publication number:
**WO 2016/114470 (21.07.2016 Gazette 2016/29)**

(54) **POMEGRANATE EXTRACT FOR ALLEVIATING WOMEN'S MENOPAUSAL SYMPTOMS, CONTAINING HIGH CONTENT OF ELLAGIC ACID**

GRANATAPFELEXTRAKT ZUR LINDERUNG DER MENOPAUSALEN SYMPTOME MIT HOHEM ANTEIL VON ELLAGISCHER SÄURE

EXTRAIT DE GRENADE PERMETTANT DE SOULAGER LES SYMPTÔMES DE LA MÉNOPAUSE CHEZ LA FEMME, CONTENANT UNE TENEUR ÉLEVÉE D'ACIDE ELLAGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2015 US 201514596778**

(43) Date of publication of application:
**22.11.2017 Bulletin 2017/47**

(73) Proprietors:
• **HLscience Co., Ltd**
  **Gyeonggi-do 18329 (KR)**
• **Lee, Hae-Yeon**
  **Uiwang-si, Gyeonggi-do 16000 (KR)**

(72) Inventor: **LEE, Hae-Yeon**
**Uiwang-si**
**Gyeonggi-do 16000 (KR)**

(74) Representative: **MERH-IP Matias Erny Reichl Hoffmann**
**Patentanwälte PartG mbB**
**Paul-Heyse-Strasse 29**
**80336 München (DE)**

(56) References cited:
**KR-A- 20110 113 122      KR-A- 20150 115 502**
**US-A1- 2013 028 994      US-A1- 2015 125 556**

• **DATABASE WPI Week 201169 Thomson Scientific, London, GB; AN 2011-M88262 XP002783091, & WO 2011/126324 A2 (HEALTH-LOVE CO LTD) 13 October 2011 (2011-10-13)**
• **LANSKY E P ET AL: "Punica granatum (pomegranate) and its potential for prevention and treatment of inflammation and cancer", JOURNAL OF ETHNOPHARMACOLOGY, ELSEVIER IRELAND LTD, IE, vol. 109, no. 2, 19 January 2007 (2007-01-19), pages 177-206, XP026140166, ISSN: 0378-8741, DOI: 10.1016/J.JEP.2006.09.006 [retrieved on 2006-12-22]**
• **KYUNG HU KIM ET AL: "The anti-climacterium effects of red clover dry extracts combined with pomegranate concentration powder in ovariectomized rats", JOURNAL OF SOCIETY OF PREVENTIVE KOREAN MEDECINE, vol. 18, no. 2, 1 August 2014 (2014-08-01), pages 133-145, XP55359509,**
• **MORI-OKAMOTO, JUNKO ET AL.: 'Pomegranate Extract improves a Depressive State and Bone Properties in Menopausal Syndrome Model Ovariectomized Mice' JOURNAL OF ETHNOPHARMACOLOGY vol. 92, 01 January 2004, pages 93 - 101, XP002497183 DOI: 10.1016/J.JEP.2004.02.006**

(Cont. next page)

- KIM, H. H. ET AL.: 'Estrogenic Activity and Physiological Active Components of Iranian Black Pomegranate Seed Extracts' INDUSTRIAL FOOD ENGINEERING vol. 11, no. 4, 2007, pages 305 - 312, XP009504423

**Description**

TECHNICAL FIELD

The present disclosure relates to a pomegranate pulp extract, a health functional food comprising the same, and an effect of the pomegranate pulp extract on relief of women's menopausal symptoms.

BACKGROUND ART

Estrogen, one of the endocrine hormones, is synthesized in vivo from cholesterol and is secreted mainly from ovarian follicles and corpora lutea. When gonadotropins secreted from the anterior pituitary gland transmit signals to reproductive organs, follicle stimulating hormone (FSH) and luteinizing hormone (LH) regulating the menstrual cycle and enabling pregnancy are secreted and act on the ovaries, where estrogen and progesterone are secreted. Estrogen is a generic name for a group of hormones, including estradiol, estrone, and estriol.

Ovaries in premenopausal women are main sources of estrogen. As women reach menopause, their aged ovaries are less likely to respond to hypophyseal gonadotropins (follicle stimulating hormone and luteinizing hormone), leading to a further shortening of the early follicular phase. As a result, the menstrual cycle and the ovulatory phase are more shortened, the production of progesterone is reduced, and the cycle is more irregular. Consequently, the ovarian follicles do not respond to hypophyseal gonadotropins, thus failing to produce estrogen any further. The reduced secretion of estrogen can have major effects on a woman's body, including the genitourinary system. Further, when ovulation stops, this leads to menopause, which signifies the end of menstruation, or the hormone reduction before menopause leads to the occurrence of menopausal symptoms.

The rapidly reduced estrogen in postmenopausal women results in significantly increased dangers of psychological and emotional symptoms such as, for example, fatigue, excitement, sleeplessness, poor concentration, depression, memory loss, headache, anxiety, and nervousness or microcardia. Other possible dangers include fatigue and excitement resulting from sleep disturbance caused by recurrent facial flushing, intermittent dizziness, paresthesia, tachycardia and pyknocardia, nausea, constipation, diarrhea, arthralgia, myalgia, cold hands and feet, and weight gain. Further, postmenopausal women are very susceptible to osteoporosis and cardiovascular diseases, including heart diseases, hypertension, and apoplexy, which are associated with an increase in mortality (Kalin MF & Zumoff B. Steroids 55:330-352, 1990). Furthermore, changes in integumentary and genitourinary systems are caused, and the possibility of the incidence of autoimmune diseases, cataract, and colorectal cancer is increased.

The most effective known method for relieving menopausal symptoms, such as facial flushing, is to administer estrogen to women in need of estrogen supplementation.

However, long-term administration of synthetic estrogen may lead to cause serious problems such as the incidence of breast cancer and uterine cancer (see Tamini RT et al. Arch intern med 166:1483-1489, 2006, Gertig DM. Menopause 13:178-184, 2006, Strom BL et al. Am J Epidemiol 164:775-586, 2006, AraJr NL & Athanazio DA. Cad Saude Publica 23:2613-2622, 2007). Many recent reliable reports have asserted that long-term administration of synthetic estrogen as a hormone replacement therapy increases the possibility of inducing breast cancer. This issue remains highly controversial in all areas of society as well as in research groups. Although the exact cause for the incidence of breast cancer is not clearly demonstrated, the risk of the incidence of breast cancer in women having undergone synthetic estrogen replacement therapy is believed to be associated with estrogen exposure dose with time.

Accordingly, considerable research efforts have been directed toward finding phytoestrogen, a type of plant-derived estrogen, as a safer substitute for synthetic estrogen used in hormone replacement therapy. Phytoestrogen refers to a non-steroidal plant compound that exerts estrogenic effects in animals, and most cereals, fruits, and vegetables known to have anticancer activity and are effective against heart diseases contain a slight amount of phytoestrogen.

That is, there is an increasing demand for a safe drug or health functional food that is effective in relieving women's menopausal symptoms without causing diseases such as breast cancer and uterine cancer.

Under these circumstances, the inventors of the present disclosure developed a health functional food including a pomegranate extract characterized by containing 0.8 to 1.8 mg/g of ellagic acid, and a patent application therefor was granted a patent under Korean Patent No. 10-1272602 on May 29, 2013. However, because a maximum concentration of ellagic acid included in the developed pomegranate extract is only 1.8 mg/g, a large amount of pomegranate extracts were often administered in expectation of a remarkable effect in relieving women's menopausal symptoms, but in this case, intake of polyphenol substances included in the pomegranate extract in large amounts causes consequential symptoms (e.g., an increase in heart bits). Therefore, there is a need for research to develop an excellent pomegranate extract which has a higher content of ellagic acid to provide an excellent relieving effect on women's menopausal symptoms even in small amounts, thereby improving the relieving effect on women's menopausal symptoms, and which may solve the problem with the limited use for some people due to unique substances in a natural product, especially polyphenol substances.

A pomegranate extract having a high ellagic acid content, and a use of the pomegranate extract are known from US 2013/028994 A1.

DISCLOSURE

Technical Problem

The present disclosure is directed to providing a pomegranate extract which is effective in relieving women's menopausal symptoms and can be administered to individuals who are sensitive to polyphenol substances, and a method of preparing the same.

Technical Solution

In order to achieve the object, the present disclosure provides a health functional food for use in relieving women's menopausal symptoms comprising, as an active ingredient, a pomegranate pulp extract according to claim 1 and a method of preparing the same according to claim 6. Preferred embodiments of the health functional food and the method are described in the respective dependent claims.

In the present disclosure, the pomegranate pulp extract contains 2.4 - 3.2 mg of ellagic acid and 10 - 14 mg of polyphenol per the total weight g of the pomegranate pulp extract.

Preferably, the present disclosure provides a pomegranate extract (pomegranate pulp extract) with an improved relieving effect on women's menopausal symptoms, containing 2.7 - 3.2 mg of ellagic acid, per the total weight g of the pomegranate extract.

When the concentration of ellagic acid is less than 1.8 mg, the relieving effect on women's menopausal symptoms is low, and when the concentration of ellagic acid is higher than 3.2 mg, there are concerns about side effects, for example, indices of liver function glutamic oxaloacetic transaminase (GOT) and glutamate pyruvate transaminase (GPT) out of the normal range.

In the present disclosure, a maximum total daily dose of the pomegranate pulp extract is less than or equal to 15 ml (preferably, less than or equal to 10 ml).

When the content of ellagic acid in the pomegranate extract increases, the relieving effect on women's menopausal symptoms may be improved, but in an attempt to increase the content of ellagic acid in the pomegranate extract, when the pomegranate extract is simply concentrated, the content of ellagic acid increases to high content, but at the same time, unique substances in a natural product, especially polyphenol substances increases in content, limiting the administration to individuals who are sensitive to polyphenol substances. Based on this discovery, the present disclosure provides a pomegranate pulp extract with a high content of ellagic acid of 2.4 - 3.2 mg per the total weight g of the pomegranate pulp extract, in which a maximum total daily dose of the pomegranate pulp extract is less than or equal to 15 ml (preferably, less than or equal to 10 ml), so the pomegranate pulp extract shows a sufficient relieving effect on women's menopausal symptoms from intake even at low doses and minimizes the polyphenol content and thus improves tolerance in individuals who are sensitive to polyphenol substances, and provides a health functional food for use in relieving women's menopausal symptoms, comprising, as an active ingredient, a pomegranate pulp extract with a high content of ellagic acid of 2.4 - 3.2 mg per the total weight g of the pomegranate pulp extract.

In the present disclosure, the menopausal symptoms include, but are not limited to, facial flushing, sudation, sleeplessness, nervousness, depression, dizziness, lack of concentration, arthralgia, headache, tachycardia, vaginal dryness, fatigue, excitement, sleeplessness, memory loss, anxiety, and an atherosclerotic vascular disease.

In the present disclosure, note that phenol is defined as a substance with one of hydrogen atoms in a benzene ring ($C_6H_6$) substituted with a hydroxyl group (-OH), and polyphenol is a substance which possesses two or more hydroxyl groups, and it has been reported that polyphenol has a positive effect such as an antioxidant function, but excessive polyphenol intake causes side effects such as, for example, the risk of liver damage or kidney damage.

In the present disclosure, sensitivity to polyphenol refers to a higher risk of side effects after polyphenol intake than a normal individual, and applies to individuals with a potential or ongoing risk of menopausal symptoms.

The pomegranate pulp extract according to the present disclosure has a sufficient relieving effect on women's menopausal symptoms while minimizing the polyphenol content to 10 mg - 14 mg, preferably 10 mg - 13 mg, and more preferably 10 mg - 12.5 mg, per the total weight g of the pomegranate pulp extract.

Pomegranate (Punica granatum L.) is a plant that grows naturally in Southwest Asia, India's northwest province and California, U.S.A. Pomegranates are widely cultivated in subtropical and tropical regions. From ancient times, pomegranates, particularly red pomegranates, have been known as tonic medicinal materials. Particularly, pomegranates are known to be very effective in preventing hypertension and arteriosclerosis. Pomegranates include water-soluble carbohydrates in amounts as large as 38 to 47% and various types of vitamins and minerals.

Although there are differences among production areas and harvest times of pomegranates used, but pomegranates used in the pomegranate pulp extract according to the present disclosure include pomegranates containing ellagic acid of 2.4 mg - 3.2 mg, and preferably 2.7 mg - 3.2 mg, per the total weight g of the final pomegranate extract. Preferably, pomegranates from Turkey or the United States of America or mixtures thereof may be used in the present disclosure, and for example, Turkish pomegranate varieties include, but are not limited to, Hicaznar pomegranate cv., Cekirdeksiz VI pomegranate cv., Silifke Asisi pomegranate cv., Katirbasi pomegranate cv. and Lefan pomegranate cv. Available pomegranates include pomegranates containing polyphenol of 10 mg - 14 mg, preferably 10 mg - 13 mg, and more preferably 10 mg - 12.5 mg, per the total weight g of the final pomegranate extract.

A pomegranate portion for preparing the pomegranate extract according to the present disclosure is a pomegranate pulp.

In the pomegranate extract according to the present disclosure, there was a big difference in the content of ellagic acid between the pomegranate extract according to the present disclosure and commercially available products, and particularly, when compared to a pomegranate extract disclosed in Korean Patent No. 10-1272602, not only the content of the foregoing representative ingredients of the pomegranate extract but also the content of substances such as polyphenol substances is nearly the same, but there is a big difference in the content of ellagic acid. Presumably, this difference leads to an excellent relieving effect on women's menopausal symptoms when the pomegranate extract is administered even at limited low daily doses. Also, the polyphenol content in the pomegranate extract according to the present disclosure is little different from that of the pomegranate extract disclosed in Korean Patent No. 10-1272602, and to achieve equivalent levels of menopausal symptom relieving effects, the pomegranate extract according to the present disclosure can achieve equivalent effects from lower dose administration as compared to the pomegranate extract disclosed in Korean Patent No. 10-1272602, and in turn, intake of polyphenol included in the pomegranate extract is reduced, so it is presumed that the pomegranate extract can be administered to polyphenol sensitive individuals.

Further may be provided a composition for relieving women's menopausal symptoms, comprising a pomegranate extract containing 1.8 - 3.2 mg of ellagic acid per the total weight g of the pomegranate extract, preferably a composition for relieving women's menopausal symptoms, comprising the pomegranate extract containing 10 mg - 14 mg of polyphenol per the total weight g of the pomegranate extract, allowing individuals who are sensitive polyphenol substances to administer.

It is presumed that the high content of ellagic acid in the pomegranate extract according to the present disclosure results from a difference in production area of a raw material pomegranates, the use of only pulps, and a particular preparation method as described below (for example, a concentration method and heating temperature and pressure of the pomegranate extract), but the present disclosure is not limited thereto.

The pomegranate pulp extract according to the present disclosure may be prepared by the following method. For example, first, after cleaning pomegranates, pericarps and seeds are completely removed from the pomegranates, followed by sterilization at high temperature in a short time, and starch degrading enzymes are added to degrade polysaccharides, such as starch, present in the pomegranates. Subsequently, optionally, an additive such as, for example, gelatin, silicon dioxide, bentonite, silicasol, tannin, cellulose, or potassium casseinate is added to control the turbidity, color, and viscosity of a pomegranate extract, followed by concentration by heating, yielding a pomegranate extract with a particular content of ellagic acid. Additionally, the method may further comprise a filtering process between the respective steps, for example, at least one step of after the peeling and deseeding step and before the high temperature sterilization step, after the step for treating with the starch degrading enzymes and before the concentration step, and after the concentration step.

More specifically, the pomegranate pulp extract containing 2.4 - 3.2 mg of ellagic acid and 10 - 14 mg of polyphenol per the total weight g of the pomegranate pulp extract may be prepared by the method including the following steps:

S1) peeling and deseeding pomegranates including Hicaznar pomegranate cv., Cekirdeksiz VI pomegranate cv., Silifke Asisi pomegranate cv., Katirbasi pomegranate cv., Lefan pomegranate cv. or mixtures thereof to obtain only pomegranate pulps;
S2) sterilizing the pomegranate pulps at 100 - 105°C for 50 - 80 seconds and cooling to 48 - 55°C;
S3) treating the cooled pomegranate pulps with starch degrading enzymes at 48 - 55°C; and
S4) concentrating the crushed pomegranate pulps by heating with elevated temperature and elevated pressure of 70 - 100°C and 400 - 850 mbar at least twice in a sequential order, and concentrating by heating with reduced temperature and reduced pressure of 40 - 80°C and 100 - 350 mbar at least once.

Optionally, the preparation method may further include a filtering step in at least one of after S1 and before S2, after S3 and before S4, and after S4.

A more detailed description of each step is provided as below.

S1) peeling and deseeding pomegranates including Hicaznar pomegranate cv., Cekirdeksiz VI pomegranate cv.,

EP 3 246 040 B1

Silifke Asisi pomegranate cv., Katirbasi pomegranate cv., Lefan pomegranate cv. or mixtures thereof to obtain only pomegranate pulps.

The present disclosure provides an extract using only pomegranate pulps without containing pomegranate pericarps and seeds. The pomegranate pericarps and seeds may cause side effects, and for example, a particular alkaloid contained in the pomegranate pericarps degrades the physical functions of humans and negatively affects the respiratory system and muscles, and addiction to alkaloid induces seizures, convulsion, and narcosis. Also, an extract from the pomegranate seeds has side effects, and for example, an allergic reaction such as tongue swelling may appear in some people after intake.

S2) sterilizing the pomegranate pulps at 100 - 105°C for 50 - 80 seconds and cooling to 48 - 55°C.

The pomegranate pulps pass through the sterilization and cooling steps in a sequential order. The sterilization is preferably performed at 100 - 105°C for 50 - 80 seconds, and more preferably, the sterilization is performed quickly for 55 - 70 seconds, and the cooling is preferably performed to 48 - 55°C.

S3) treating the cooled pomegranate pulps with starch degrading enzymes at 48 - 55°C.

The cooled pomegranate pulps undergone treatment with starch degrading enzymes. Preferably, this step may be performed at 48 - 55°C for 10 - 60 minutes, more preferably at 48 - 55°C for 20 - 40 minutes. Available starch degrading enzymes are not limited to a particular type, and include various starch degrading enzymes known in the art such as, for example, pectinase, proteinase, amylase, and cellulase, preferably pectinase.

S4) concentrating the crushed pomegranate pulps by heating with elevated temperature and elevated pressure of 70 - 100°C and 400 - 850 mbar at least twice in a sequential order, and concentrating by heating with reduced temperature and reduced pressure of 40 - 80°C and 100 - 350 mbar at least once.

The crushed pomegranate pulps undergo concentration by heating with elevated temperature and elevated pressure in a sequential order and concentration by heating with reduced temperature and reduced pressure.

Preferably, the concentration by heating may include concentration by heating with elevated temperature and elevated pressure at least twice, more preferably at least three times, and concentration by heating with reduced temperature and reduced pressure at least once, more preferably at least twice, totaling at least three times.

The concentration by heating with elevated temperature and elevated pressure is performed within the range of temperature condition of 70 - 100°C and the pressure condition of 400 - 850 mbar, with varying temperature and pressure each concentration by heating, and if the elevated temperature and the elevated pressure falls within the above temperature and pressure ranges, there is no limitation on the temperature and pressure and such variations may be included in the scope of the present disclosure. Preferably, first concentration by heating may be performed at 70 - 85°C and 400 - 550 mbar, second concentration by heating may be performed at 85 - 92°C and 550 - 750 mbar, and third concentration by heating may be performed at 92 - 100°C and 750 - 850 mbar. More preferably, first concentration by heating may be performed at 78 - 82°C and 450 - 500 mbar, second concentration by heating may be performed at 85 - 90°C and 600 - 650 mbar, and third concentration by heating may be performed at 92 - 98°C and 800 - 850 mbar.

The concentration by heating with reduced temperature and reduced pressure is performed within the range of temperature condition of 40 - 80°C and pressure condition of 100 - 350 mbar, with varying temperature and pressure each concentration by heating, and if the reduced temperature and the reduced pressure falls within the above temperature and pressure ranges, there is no limitation on the temperature and pressure and such variations may be included in the scope of the present disclosure. Preferably, fourth concentration by heating may be performed at 60 - 80°C and 250 - 350 mbar and fifth concentration by heating may be performed at 40 - 60°C and 100 - 250 mbar. More preferably, fourth concentration by heating may be performed at 65 - 72°C and 300 - 330 mbar and fifth concentration by heating may be performed at 45 - 55°C and 100 - 150 mbar.

Further provided may be a composition for relieving women's menopausal symptoms, comprising, as an active ingredient, a pomegranate extract containing 1.8 - 3.2 mg of ellagic acid per the total weight g of the pomegranate extract, in which a maximum total daily dose of the pomegranate extract is less than or equal to 15 ml (preferably, less than or equal to 10 ml). The composition for relieving women's menopausal symptoms has a wide range of applications, for example, a pharmaceutical composition or a health functional food.

The present disclosure provides a health functional food for use in relieving women's menopausal symptoms, comprising, as an active ingredient, the pomegranate extract containing 2.4 - 3.2 mg of ellagic acid per the total weight g of the pomegranate extract, in which a maximum total daily dose of the pomegranate extract is less than or equal to 15 ml (preferably, less than or equal to 10 ml).

The health functional food according to the present disclosure includes, as an active ingredient, a pomegranate extract containing a high concentration of ellagic acid and an equivalent amount or smaller amount of unique substances in a natural product, especially polyphenol substances, as compared to traditional pomegranate extracts. Thus, when a maximum total daily dose of the pomegranate extract is less than or equal to 15 ml (preferably, less than or equal to 10 ml), a significantly excellent relieving effect on women's menopausal symptoms is provided and it can be thus administered to individuals who are sensitive to polyphenol substances.

Furthermore, in an embodiment of the present disclosure, when the pomegranate pulp extract according to the present disclosure is administered at daily doses of 15 ml, human health indices (for example, red blood cell (RBC) count, blood urea nitrogen (BUN) level, aspartate aminotransferase (AST) level, alanine aminotransferase (ALT) level, creatinine level, glucose level, and S-G value) are all within the normal ranges after administration, so the relieving effect on menopausal symptoms may be achieved in a safe manner without side effects in human body.

The health functional food according to the present disclosure may be fractionally packaged in an individual container in a single dose, and the single dose may include 15 ml - 1 ml of the pomegranate extract, preferably any one dose of 15 ml, 14 ml, 13 ml, 12 ml, 11 ml, 10 ml, 5 ml, 4 ml, 3 ml, 2 ml, and 1 ml.

In addition to the pomegranate extract, the health functional food according to the present disclosure may further include Paeonia japonica, Cornus officinalis, Acanthopanax senticosus, Ganoderma lucidium, the stem bark of Fraxinus rhynchophylla, Eucommia ulmoides, Angelica gigas, Gardenia jasminoides, Astragalus membranaceus, malt, trifoliate orange, vitamin C, fructooligosaccharides, stevioside, purified water, and maltodextrin, alone or in combination, without departing from the scope of the present disclosure, but other pharmacologically active ingredients and/or additives further included in the health functional food of the present disclosure are not limited to the foregoing examples.

For example, the health functional food according to the present disclosure may include water-soluble vitamins, such as thiamine (vitamin $B_1$), riboflavin, ascorbic acid, niacin, and vitamin $B_6$; fatty acids, such as myristic acid, palmitic acid, stearic acid, oleic acid, and linoleic acid; weak acids, such as glycolic acid and acetic acid; and amino acids, such as eight essential amino acids, i.e., threonine, valine, methionine, isoleucine, leucine, phenylalanine, tryptophan and lysine, aspartic acid, serine, glutamic acid, proline, glycine, alanine, cysteine, tyrosine, histidine, and arginine.

Provided may be a method for improving the effect of a pomegranate extract on relief of women's menopausal symptoms, and preferably, the method may include adjusting the content of ellagic acid to 1.8 - 3.2 mg, more preferably 2.4 mg - 3.2 mg, and even more preferably 2.7 mg - 3.2 mg, per the total weight g of the pomegranate extract. Preferably, the method may include adjusting the polyphenol content to 10 mg - 14 mg, more preferably 10 mg - 13 mg, and most preferably 10 mg - 12.5 mg, per the total weight g of the pomegranate extract.

Advantageous Effects

According to the present disclosure, an excellent relieving effect on women's menopausal symptoms is expected from intake even at low daily doses, thus allowing individuals who are sensitive to polyphenol substances to administer.

BEST MODE FOR CARRYING OUT THE INVENTION

Hereinafter, the present disclosure will be explained in detail with reference to the examples to help the understanding of the present disclosure. However, these examples may be embodied in various different forms and should not be construed as limiting the scope of the present disclosure. The examples are provided to more fully explain the present disclosure to those having ordinary knowledge in the art to which the present disclosure pertains.

**<Example 1> Preparation of pomegranate extracts with high content of ellagic acid**

First, any foreign matter was removed from 1,000 kg of pomegranates, and fruits not damaged were chosen and cleaned. The sorted fruits were cut, peeled, and deseeded using compression, yielding 450 kg of pomegranate pulps. After filtering, the pomegranate pulps were sterilized at 1000 - 105°C for 60 seconds and cooled to 48 - 55°C again. Pectinase was added in an amount of 70-100 ml per 1,000 L of the pomegranate juice, allowing starch degradation at 48 - 55°C for 30 minutes. Subsequently, to maintain the turbidity and color and attain the viscosity suitable for administration, bentonite was added in an amount of 900 g per 10,000 L of the pomegranate juice, followed by stirring at 48 - 55°C for 10 minutes. Subsequently, after 1.5 mm and 1 mm vacuum filtration, concentration by heating was performed (in a sequential order, up to 12 brix at 80°C and 475 mbar, up to 17 brix at 87°C and 626 mbar, up to 31 Brix at 95°C and 847 mbar, up to 43 Brix at 70°C and 312 mbar, and up to 65 Brix at 49°C and 118 mbar), and 1.5 mm filtration was performed again to prepare a pomegranate extract containing 1.8 - 3.2 mg/g of ellagic acid. The pomegranate extract underwent aseptic filling and was individually packaged as 10 ml. The same process was performed iteratively six times.

**<Example 2> Evaluation of ellagic acid content in pomegranate extract**

The content of ellagic acid in the pomegranate extract was measured using high-performance liquid chromatography (HPLC). SP C18 UG 120 (4.6 mm × 50 mm, 5 μm) column was used with mobile phase of a mixture of 0.85% phosphoric acid/water and methanol (6:4) and pure methanol in gradient elution mode, and ellagic acid was detected at a wavelength of 370 nm using a UV detector. As a result, it was found that a mean content of ellagic acid in the pomegranate extract prepared according to EXAMPLE 1 was 1.8 - 3.2 mg/g.

Along with this, the content of ellagic acid in three commercially available products, pomegranate extracts prepared according to Korean Patent No. 10-1272602, and the pomegranate extracts prepared according to EXAMPLE 1 was evaluated by the above mentioned method, and their results were shown in Table 1. For each product, six samples with different lot numbers were each bought and evaluated to determine the content of ellagic acid.

[Table 1]

| (Unit: mg/g) | Product A | Product B | Product C | No. 10-1272602 | Inventive Extract | |
|---|---|---|---|---|---|---|
| Sample No. 1 | 0.21 | 0.39 | 0.27 | 0.80 | 1.80 | |
| Sample No. 2 | 0:18 | 0.22 | 0.40 | 0.87 | 2.49 | |
| Sample No. 3 | 0.46 | 0.08 | 0.21 | 1.01 | 2.67 | |
| Sample No. 4 | 0.19 | 0.13 | 0.27 | 1.40 | 2.82 | |
| Sample No. 5 | 0.34 | 0.21 | 0.35 | 1.28 | 2.90 | |
| Sample No. 6 | 0.32 | 0.19 | 0.37 | 1.09 | 3.20 | |
| Ellagic acid content range | 0.18~0.46 | 0.08~0.39 | 0.21~0.40 | 0.08~1.40 | 1.80~3.20 | |

In Table 1, Product A is a pomegranate extract product available from Shadaab Co., Product B is a pomegranate extract product available from Pashapour Trading Co., and Product C is a pomegranate extract product available from Noosh Iran Co. Furthermore, No. 10-1272602 stands for a pomegranate extract prepared according to the preparation method described in Korean Patent No. 10-1272602.

As shown in Table 1, it can be seen that the content of ellagic acid in the pomegranate extract according to the present disclosure is very high as compared to the other commercially available products as well as the pomegranate extract according to Korean Patent No. 10-1272602 (sugar content: 65 brix).

Particularly, when comparing based on the content of ellagic acid, to administer the same amount of ellagic acid in the form of a pomegranate extract, the dose of the pomegranate extract according to Korean Patent No. 10-1272602 is about twice higher than that of the pomegranate extract of the present disclosure.

<Example 3> Evaluation of total polyphenol in pomegranate extract

The content of polyphenol in the pomegranate extract was calculated by dissolving the sample in distilled water, performing ultrasonic extraction, causing a reaction with a color reagent, and metering using a spectrophotometer.

10 mg of tannic acid was precisely metered and put in a 100 mL volumetric flask which was filled with distilled water up to a gauge mark, and this standard undiluted solution was distilled with distilled water to an optimum concentration to make a standard solution. A test solution was prepared by metering 100 mg of the pomegranate extract prepared according to EXAMPLE 1 and putting it in a 100 mL volumetric flask where distilled water was introduced up to a gauge mark, followed by ultrasonic treatment to dissolve it. After 7.5 mL of distilled water was poured into a test tube and 1 mL of each of the standard solution and the test solution was poured into the same test tube, 0.5 mL of Folin-denis reagent and 1 mL of 35% sodium carbonate was added certainly in a sequential order and mixed with each other, and after being left in darkness for one hour, absorbance was measured at 760 nm using UV/Visible Spectrophotometer. A calibration curve of the standard material was plotted using a difference between absorbance of a standard material for each concentration and absorbance of each blank test, and the total polyphenol content in the sample was calculated by applying, to the calibration curve, a difference between absorbance of the pomegranate extract prepared according to EXAMPLE 1 and blank test absorbance of the pomegranate extract prepared according to EXAMPLE 1.

$$\text{Total polyphenol content (mg/g)} = (A \times B \times C) / D$$

A: Total amount of test solution (mL)

B: Dilution factor

C: Total polyphenol concentration in test solution (mg/mL)

D: Sample size (g or mL)

The total polyphenol content in the pomegranate extracts prepared according to Korean Patent No. 10-1272602 was evaluated in the same way as the above mentioned method.

The evaluation results were shown in Table 2.

[Table 2]

| (Unit: mg/g) | No. 10-1272602 | Inventive Extract |
|---|---|---|
| Sample No. 1 | 11.79 | 12.59 |
| Sample No. 2 | 10.59 | 12.67 |
| Sample No. 3 | 12.35 | 11.72 |
| Sample No. 4 | 12.89 | 13.21 |
| Sample No. 5 | 13.12 | 11.03 |
| Sample No. 6 | 11.02 | 10.89 |
| Average value of total polyphenols | 11.96±1.01 | 12.01±0.94 |

As shown in Table 2, it was found that the total polyphenol content in the pomegranate extract of the present disclosure was similar to that of the pomegranate extract prepared according to Korean Patent No. 10-1272602.

From a combination of these results and the ellagic acid content results in Table 1, the effect of a pomegranate extract with a high content of ellagic acid amounting to 1.80~3.20 mg/g in the pomegranate extract of the present disclosure does not result from mere concentration of a larger amount of pomegranate extracts.

**<Example 4> Evaluation of the extent to which menopausal symptoms and climacteric symptoms are relieved**

The extent to which the pomegranate pulp extract with a high content of ellagic acid according to the present disclosure relieves women's menopausal symptoms was evaluated by the following method.

Subjects

For a screening test, volunteers were publicly selected from women, aged 40-60, with 12 months or more of amenorrhoea and an FSH value less than or equal to 40 mlU/ml and Kupperman index higher than or equal to 20. The details of the test were notified, and after being fully understood, the volunteers determined to participate in the test and gave a written consent to follow instructions, and the test was then conducted.

One week prior to administration of the test extract, basic information of the volunteers, such as demographic data and past medical history, was recorded, and after the nutritive conditions, integumentary/mucosal system, otolaryngology system, cardiovascular system, urogenital system, respiratory system, metabolic/endocrine system, digestive system, musculoskeletal system, nervous/mental system, and other physical conditions were medically inspected, normal and abnormal findings were recorded.

According to general exclusion criteria for Kupperman testing, the following subjects were excluded: subjects who suffered from psychogenic menopausal disorders, who had undergone hormone replacement therapy before at least 6 months of testing or were receiving hormone replacement therapy at the time of testing, who had amenorrhoea after bilateral oophorectomy or hysterectomy within the last 12 months, who suffered from heart diseases (e.g., heart failure, angina, and myocardial infarction), who suffered from uncontrollable hypertension, who suffered from malignant tumor, narrow-angle glaucoma or lung disease, and who suffered from severe renal or hepatic dysfunction.

Test methods

The test was conducted for a total of 8 weeks. A total of 60 subjects (30 subjects in each group) consisted of a control group (6.5 g of fructooligosaccharide, 1 g of fructose, 0.4 g of pigment in red cabbage, 0.5 g of citric acid, and 0.1 g of pomegranate flavor in purified water) and an experimental group for the pomegranate extract according to the present disclosure (1.8-3.2 mg/g of ellagic acid).

After finishing a baseline test, the total of 60 subjects were divided into an experimental group and a control group at random. Firstly, a 4 weeks' amount of test products were provided to all the subjects. The experimental group was required to take the pomegranate extract at a daily dose of 10 ml/day. 4 weeks after administration, the volunteered were instructed to store and return the pouches after administration to confirm whether they exactly administered, and the vital signs, adverse events, and concomitant drugs of the volunteers were examined and laboratory examinations were performed (indicating 'second visit' test results in the following table). Secondly, a 4 weeks' amount of test products were provided to all the subjects. The experimental group was required to take the pomegranate extract at a daily dose of 10 ml/day. Likewise, after 4 weeks passed, examinations were performed (indicating third visit' test results in the following table).

Evaluation and results of Kupperman index. Menopause rating scale (MRS), FSH, and estradiol (E2)

For first effectiveness evaluation, Kupperman index generally used to evaluate menopausal symptoms and climacteric symptoms was checked. Immediately before and after administration, an investigator examined the subjects directly, evaluated collectively the occurrence, intensity, and frequency of symptoms for Kupperman index parameters, and recorded the scores on the questionnaire, and through this, the effect of the pomegranate extract with a high content of ellagic acid according to the present disclosure was evaluated.

For second effectiveness evaluation, MRS, FSH, and estradiol (E2) variances were checked.

The effectiveness evaluation was conducted before/after administration and each visit, and a test for homogeneity was analyzed by an independent t-test, an effect test before/after administration was analyzed by an independent t-test at a significance level of 5% or less, and as a result of the test for homogeneity, variances not equal were adjusted for covariates and analyzed using ANCOVA. Furthermore, effect tests per visit were analyzed using a repeated measures ANOVA (RM ANOVA) at a significance level of 5% or less, and as a result of the test for homogeneity, variances not equal were analyzed using a repeated measures ANOVA (RM ANOVA).

[Table 3]

| | Pomegranate concentrate group (n=30) | | | | Placebo group (n=30) | | | | $P$-value[1] |
|---|---|---|---|---|---|---|---|---|---|
| | Screening | Second visit | Third visit | $P$-value[1] | Screening | Second visit | Third visit | $P$-value[1] | |
| KI (score) | 36.73±5.90 | 22.30±9.55 | 14.67±6.88 | <.0001 | 34.00±7.05 | 28.83±7.76 | 26.52±8.46 | <.0001 | <.0001 |

Values are presented as mean±SD or number.
[1] Analyzed by Linear mixed model for repeated measure data

[Table 4]

| | Pomegranate concentrate group (n=30) | | | Placebo group (n=30) | | | P-value[2] |
|---|---|---|---|---|---|---|---|
| | Screening | Third visit | P-value[1] | Screening | Third visit | P-value[1] | |
| KI (score) | 36.73±5.90 | 14.67±6.88 | <.0001 | 34.00±7.05 | 26.52±8.46 | <.0001 | <.0001 |

Values are presented as mean±SD or number.
[1] Analyzed by Paired t-test
[2] Analyzed by Independent t-test

[Table 5]

| | Pomegranate concentrate group (n=30) | | | | Placebo group (n=30) | | | | *P*-value[1] |
|---|---|---|---|---|---|---|---|---|---|
| | Screening | Second visit | Third visit | *P*-value[1] | Screening | Second visit | Third visit | *P*-value[1] | |
| MRS (score) | 26.13±6.14 | 16.00±6.99 | 10.20±5.03 | <.0001 | 25.90±7.55 | 21.07±6.49 | 18.45±5.82 | <.0001 | <.0001 |
| FSH (mIU/mL) | 84.08±30.60 | 81.98±27.30 | 75.21±23.81 | 0.003 | 91.36±20.64 | 87.57±20.61 | 81.68±17.55 | <.0001 | 0.803 |
| Estradiol (pg/mL) | 6.43±4.68 | 5.19±5.80 | 6.25±9.04 | 0.537[2] | 8.37±6.73 | 5.81±4.74 | 6.18±5.94 | 0.171 | 0.618 |

Values are presented as mean±SD or number.

[1] Analyzed by Linear mixed model for repeated measure data

[2] Analyzed by repeated measured ANOVA

[Table 6]

| | Pomegranate concentrate group (n=30) | | | Placebo group (n=30) | | | P-value[2] |
|---|---|---|---|---|---|---|---|
| | Screening | Third visit | P-value[1] | Screening | Third visit | P-value[1] | |
| MRS (score) | 26.13±6.14 | 10.20±5.03 | <.0001 | 25.90±7.55 | 18.45±5.82 | <.0001 | <.0001 |
| FSH (mIU/mL) | 84.08±30.60 | 75.21±23.81 | 0.001 | 91.36±20.64 | 81.68±17.55 | <.0001 | 0.549 |
| Estradiol (pg/mL) | 6.43±4.68 | 6.25±9.04 | 0.893 | 8.37±6.73 | 6.18±5.94 | 0.214 | 0.352 |

Values are presented as mean±SD or number.
[1] Analyzed by Paired t-test
[2] Analyzed by Independent t-test

As shown in the results of Tables 3 - 6, the pomegranate pulp extract with a high content of ellagic acid according to the present disclosure was far better in the relieving effect on menopausal symptoms than the control.

More specifically, as shown in Tables 3 - 4, it can be seen that the Kupperman indices of the subjects were reduced by 50% or more after 8 weeks passed. Moreover, as shown in Tables 5 - 6, it can be seen that MRS also significantly reduced after 8 weeks passed, and FSH and estradiol concentration remarkably reduced as well.

**<COMPARATIVE EXAMPLE 1> Comparison to Korean Patent No. 10-1272602 pomegranate extract**

The pomegranate extract (sugar content: 65 brix, ellagic acid: 0.80~1.40 mg/g) was prepared according to the method described in Korean Patent No. 10-1272602, which was used as an experimental group, and Kupperman index evaluation was conducted by the same method as described in EXAMPLE 3, with the pomegranate extract intake at a daily dose of 40 ml/day.

After 8 weeks passed, as a result of evaluating a change in total Kupperman index score between before and after intake of the pomegranate extract, KI (total score) was found about 15.08.

As a result, it was found that the Kupperman indices evaluated after administering the pomegranate extract according to EXAMPLE 1 and the pomegranate extract of Korean Patent No. 10-1272602 for the same duration showed similar levels, but when considering a daily dose of the pomegranate extract according to EXAMPLE 1 is 10 ml/day while a daily dose of the pomegranate extract of Korean Patent No. 10-1272602 is 40 ml/day, it is required to administer the pomegranate extract daily in amounts larger four times to achieve equivalent levels of menopausal symptom relieving effects.

In combining the results of EXAMPLE 3 and COMPARATIVE EXAMPLE 1 with Table 2 showing the evaluation results of the polyphenol content, because polyphenol of a similar weight is contained in the pomegranate extract of the present disclosure and the pomegranate extract prepared according to Korean Patent No. 10-1272602, when the pomegranate extract prepared according to Korean Patent No. 10-1272602 is administered at doses of 40 ml/day, polyphenol is administered together in amounts larger about four times to achieve equivalent levels of menopausal symptom relieving effects, as compared to the pomegranate extract of the present disclosure administered at doses of 10 ml/day for the same duration. As a result, to achieve equivalent levels of menopausal symptom relieving effects as compared to existing pomegranate extracts including the pomegranate extract of Korean Patent No. 10-1272602, the pomegranate extract according to the present disclosure can achieve equivalent effects from lower dose administration, and in turn, intake of polyphenol included in the pomegranate extract is reduced, so it is found that the pomegranate extract can be administered to polyphenol sensitive individuals.

MODE FOR CARRYING OUT THE INVENTION

Hereinafter, it should be understood that the scope of the present disclosure is not construed as being limited to the following description.

The present disclosure relates to a health functional food for use in relieving women's menopausal symptoms, comprising, as an active ingredient, a pomegranate extract containing 2.4 - 3.2 mg of ellagic acid per the total weight g of the pomegranate extract.

The present disclosure relates to a health functional food for use in relieving women's menopausal symptoms, comprising, as an active ingredient, a pomegranate extract containing 2.4 - 3.2 mg of ellagic acid per the total weight g of the pomegranate extract, in which a maximum total daily dose of the pomegranate extract is less than or equal to 15 ml.

More specifically, the health functional food may be fractionally packaged in an individual container in any one single dose of the pomegranate extract of 15 ml, 14 ml, 13 ml, 12 ml, 11 ml, 10 ml, 5 ml, 4 ml, 3 ml, 2 ml, and 1 ml.

The pomegranate may be from Turkey, the United States of America, or mixtures thereof.

The health functional food can be administered to an individual who is sensitive to polyphenol substances.

The pomegranate extract is a pomegranate pulp extract.

The pomegranate pulp extract contains 10 - 14 mg of polyphenol per the total weight g of the pomegranate pulp extract.

The present disclosure provides a method of preparing a pomegranate pulp extract containing 2.4 - 3.2 mg of ellagic acid per the total weight g of the pomegranate pulp extract, the method including the steps of:

S1) peeling and deseeding pomegranates including Hicaznar pomegranate cv., Cekirdeksiz VI pomegranate cv., Silifke Asisi pomegranate cv., Katirbasi pomegranate cv., Lefan pomegranate cv. or mixtures thereof to obtain only pomegranate pulps;

S2) sterilizing the pomegranate pulps at 100 - 105°C for 50 - 80 seconds and cooling to 48 - 55°C;

S3) treating the cooled pomegranate pulps with starch degrading enzymes at 48 - 55°C; and

S4) concentrating the crushed pomegranate pulps by heating with the elevated temperature and elevated pressure of 70 - 100°C and 400 - 850 mbar at least twice in a sequential order, and concentrating by heating with the reduced temperature and reduced pressure of 40 - 80°C and 100 - 350 mbar at least once.

More specifically, the preparation method may further include a filtering step in at least one of after S1 and before S2, after S3 and before S4, and after S4 S1.

The pomegranate pulp extract can be administered to an individual who is sensitive to polyphenol substances.

The S4 may include concentrating by heating with 400 - 550 mbar at 70 - 85°C, concentrating by heating with 550 - 750 mbar at 85 - 92°C, concentrating by heating with 750 - 850 mbar at 92 - 100°C, concentrating by heating with 250 - 350 mbar at 60 - 80°C, and concentrating by heating with 100 - 250 mbar at 40 - 60°C.

The pomegranate pulp extract contains 10 - 14 mg of polyphenol per the total weight g of the pomegranate pulp extract.

Further, the present disclosure relates to a health functional food for use in relieving women's menopausal symptoms, comprising the pomegranate pulp extract prepared by the above preparation method as an active ingredient, in which a maximum total daily dose of the pomegranate pulp extract is less than or equal to 15 ml.

Further, a pharmaceutical composition for relieving women's menopausal symptoms, may comprise, as an active ingredient, a pomegranate extract containing 1.8 - 3.2 mg of ellagic acid per the total weight g of the pomegranate extract.

Further, a pharmaceutical composition for relieving women's menopausal symptoms, may comprise, as an active ingredient, a pomegranate extract containing 1.8 - 3.2 mg of ellagic acid per the total weight g of the pomegranate extract, in which a maximum total daily dose of the pomegranate extract is less than or equal to 15 ml.

INDUSTRIAL APPLICABILITY

According to the present disclosure, an excellent relieving effect on women's menopausal symptoms is expected from intake even at low daily doses, thus allowing individuals who are sensitive to polyphenol substances to administer.

**Claims**

1. A health functional food for use in relieving women's menopausal symptoms, comprising, as an active ingredient, a pomegranate pulp extract containing 2.4 - 3.2 mg of ellagic acid and 10 - 14 mg of polyphenol per the total weight g of the pomegranate pulp extract.

2. The health functional food for use in relieving women's menopausal symptoms according to claim 1, wherein a maximum total daily dose of the pomegranate pulp extract is less than or equal to 15 ml.

3. The health functional food for use in relieving women's menopausal symptoms according to claim 2, wherein the health functional food is fractionally packaged in an individual container in any one single dose of the pomegranate pulp extract of 15 ml, 14 ml, 13 ml, 12 ml, 11 ml, 10 ml, 5 ml, 4 ml, 3 ml, 2 ml, and 1 ml.

4. The health functional food for use in relieving women's menopausal symptoms according to claim 1 or 2, wherein the pomegranate includes Hicaznar pomegranate cv., Cekirdeksiz VI pomegranate cv., Silifke Asisi pomegranate cv., Katirbasi pomegranate cv., Lefan pomegranate cv. or mixtures thereof.

5. The health functional food for use in relieving women's menopausal symptoms according to claim 1 or 2, wherein the health functional food improves tolerance in an individual who is sensitive to polyphenol substances.

6. A method of preparing a pomegranate pulp extract containing 2.4 - 3.2 mg of ellagic acid and 10 - 14 mg of polyphenol per the total weight g of the pomegranate pulp extract, the method comprising:

S1) peeling and deseeding a pomegranate including Hicaznar pomegranate cv., Cekirdeksiz VI pomegranate cv., Silifke Asisi pomegranate cv., Katirbasi pomegranate cv., Lefan pomegranate cv. or mixtures thereof to obtain only a pomegranate pulp;
S2) sterilizing the pomegranate pulp at 100 - 105°C for 50 - 80 seconds and cooling to 48 - 55°C;
S3) treating the cooled pomegranate pulp with a starch degrading enzyme at 48 - 55°C; and
S4) concentrating the crushed pomegranate pulp by heating with elevated temperature and elevated pressure of 70 - 100°C and 400 - 850 mbar at least twice in a sequential order, and concentrating by heating with reduced temperature and reduced pressure of 40 - 80°C and 100 - 350 mbar at least once.

7. The method of preparing a pomegranate pulp extract according to claim 6, wherein the method further comprises filtering in at least one of after S1 and before S2, after S3 and before S4, and after S4.

8. The method of preparing a pomegranate pulp extract according to claim 6, wherein the pomegranate pulp extract improves tolerance in an individual who is sensitive to polyphenol substances.

9. The method of preparing a pomegranate pulp extract according to claim 6, wherein the S4 comprises concentrating by heating with 400 - 550 mbar at 70

- 85°C, concentrating by heating with 550 - 750 mbar at 85 - 92°C, concentrating by heating with 750 - 850 mbar at 92 - 100°C, concentrating by heating with 250
- 350 mbar at 60 - 80°C, and concentrating by heating with 100 - 250 mbar at 40 - 60°C.

10. A health functional food for use in relieving women's menopausal symptoms, comprising the pomegranate pulp extract prepared by the preparing method of claim 6 as an active ingredient, in which a maximum total daily dose of the pomegranate pulp extract is less than or equal to 15 ml.

**Patentansprüche**

1. Funktionale Gesundheitskost zur Verwendung beim Lindern von Menopausensymptomen von Frauen, die als Wirkstoff einen Granatapfelfruchtmarkextrakt umfasst, der 2,4-3,2 mg Ellagsäure und 10-14 mg Polyphenol pro Gesamtgewicht g des Granatapfelfruchtmarkextrakts enthält.

2. Funktionale Gesundheitskost zur Verwendung beim Lindern von Menopausensymptomen von Frauen nach Anspruch 1, wobei eine maximale tägliche Gesamtdosis des Granatapfelfruchtmarkextrakts geringer als oder gleich 15 ml ist.

3. Funktionale Gesundheitskost zur Verwendung beim Lindern von Menopausensymptomen von Frauen nach Anspruch 2, wobei die funktionale Gesundheitskost in einem individuellen Behälter in irgendeiner Einzeldosis des Granatapfelfruchtmarkextrakts von 15 ml, 14 ml, 13 ml, 12 ml, 11 ml, 10 ml, 5 ml, 4 ml, 3 ml, 2 ml und 1 ml fraktioniert verpackt ist.

4. Funktionale Gesundheitskost zur Verwendung beim Lindern von Menopausensymptomen von Frauen nach Anspruch 1 oder 2, wobei der Granatapfel Hicaznar-Granatapfel cv., Cekirdeksiz-VI-Granatapfel cv. Silifke-Asisi-Granatapfel cv. Katirbasi-Granatapfel cv. Lefan-Granatapfel cv. oder Gemische davon umfasst.

5. Funktionale Gesundheitskost zur Verwendung beim Lindern von Menopausensymptomen von Frauen nach Anspruch 1 oder 2, wobei die funktionale Gesundheitskost die Toleranz bei einer Person verbessert, die gegen Polyphenolsubstanzen empfindlich ist.

6. Verfahren zur Zubereitung eines Granatapfelfruchtmarkextrakts, der 2,4-3,2 mg Ellagsäure und 10-14 mg Polyphenol pro Gesamtgewicht g des Granatapfelfruchtmarkextrakts enthält, wobei das Verfahren umfasst:

S1) Schälen und Entkernen eines Granatapfels, einschließlich Hicaznar-Granatapfel cv., Cekirdeksiz-VI-Granatapfel cv. Silifke-Asisi-Granatapfel cv. Katirbasi-Granatapfel cv. Lefan-Granatapfel cv. oder Gemischen davon, um nur ein Granatapfelfruchtmark zu erhalten;

S2) Sterilisieren des Granatapfelfruchtmarks bei 100-105 °C für 50-80 Sekunden und Kühlen auf 48-55 °C;

S3) Behandeln des gekühlten Granatapfelfruchtmarks mit einem Stärke abbauenden Enzym bei 48-55 °C; und

S4) Konzentrieren des zerkleinerten Granatapfelfruchtmarks durch mindestens zweimaliges Erhitzen mit erhöhter Temperatur und erhöhtem Druck von 70-100 °C und 400-850 mbar in einer sequentiellen Reihenfolge und Konzentrieren durch mindestens einmaliges Erhitzen mit verringerter Temperatur und verringertem Druck von 40-80 °C und 100-350 mbar.

7. Verfahren zur Zubereitung eines Granatapfelfruchtmarkextrakts nach Anspruch 6, wobei das Verfahren ferner das Filtern nach S1 und vor S2, nach S3 und vor S4 und/oder nach S4 umfasst.

8. Verfahren zur Zubereitung eines Granatapfelfruchtmarkextrakts nach Anspruch 6, wobei der Granatapfelfruchtmarkextrakt die Toleranz bei einer Person verbessert, die gegen Polyphenolsubstanzen empfindlich ist.

9. Verfahren zur Zubereitung eines Granatapfelfruchtmarkextrakts nach Anspruch 6, wobei S4 das Konzentrieren durch Erhitzen mit 400 - 550 mbar bei 70 - 85 °C, das Konzentrieren durch Erhitzen mit 550-750 mbar bei 85-92 °C, das Konzentrieren durch Erhitzen mit 750-850 mbar bei 92-100 °C, das Konzentrieren durch Erhitzen mit 250-350 mbar bei 60-80 °C und das Konzentrieren durch Erhitzen mit 100-250 mbar bei 40-60 °C umfasst.

10. Funktionale Gesundheitskost zur Verwendung beim Lindern von Menopausensymptomen von Frauen mit dem durch das Zubereitungsverfahren nach Anspruch 6 zubereiteten Granatapfelfruchtmarkextrakt als Wirkstoff, wobei eine maximale tägliche Gesamtdosis des Granatapfelfruchtmarkextrakts geringer als oder gleich 15 ml ist.

**Revendications**

1. Aliment fonctionnel diététique destiné à une utilisation permettant de soulager les symptômes de la ménopause chez la femme, comprenant, à titre d'ingrédient actif, un extrait de pulpe de grenade contenant 2,4 à 3,2 mg d'acide ellagique et 10 à 14 mg de polyphénol par g de poids total d'extrait de pulpe de grenade.

2. Aliment fonctionnel diététique destiné à une utilisation permettant de soulager les symptômes de la ménopause chez la femme selon la revendication 1, dans lequel une dose quotidienne totale maximale de l'extrait de pulpe de grenade est inférieure ou égale à 15 ml.

3. Aliment fonctionnel diététique destiné à une utilisation permettant de soulager les symptômes de la ménopause chez la femme selon la revendication 2, ledit aliment fonctionnel diététique étant conditionné de manière fractionnée dans un récipient individuel en une unique dose quelconque de l'extrait de pulpe de grenade de 15 ml, 14 ml, 13 ml, 12 ml, 11 ml, 10 ml, 5 ml, 4 ml, 3 ml, 2 ml, et 1 ml.

4. Aliment fonctionnel diététique destiné à une utilisation permettant de soulager les symptômes de la ménopause chez la femme selon la revendication 1 ou 2, dans lequel la grenade comprend la grenade cv. Hicaznar, la grenade cv. Cekirdeksiz VI, la grenade cv. Silifke Asisi, la grenade cv. Katirbasi, la grenade cv. Lefan ou des mélanges de celles-ci.

5. Aliment fonctionnel diététique destiné à une utilisation permettant de soulager les symptômes de la ménopause chez la femme selon la revendication 1 ou 2, ledit aliment fonctionnel diététique améliorant la tolérance chez un individu qui est sensible aux substances polyphénolées.

6. Méthode de préparation d'un extrait de pulpe de grenade contenant 2,4 à 3,2 mg d'acide ellagique et 10 à 14 mg de polyphénol par g de poids total d'extrait de pulpe de grenade, ladite méthode comprenant :

S1) l'épluchage et l'épépinage d'une grenade comprenant la grenade cv. Hicaznar, la grenade cv. Cekirdeksiz VI, la grenade cv. Silifke Asisi, la grenade cv. Katirbasi, la grenade cv. Lefan ou des mélanges de celles-ci, pour obtenir seulement une pulpe de grenade ;

S2) la stérilisation de la pulpe de grenade à 100 - 105°C pendant 50 à 80 secondes et le refroidissement à 48 - 55°C ;

S3) le traitement de la pulpe de grenade refroidie avec une enzyme dégradant l'amidon à 48 - 55°C ; et
S4) la concentration de la pulpe de grenade broyée par chauffage à une température élevée et une pression élevée de 70 - 100°C et 400 - 850 mbar au moins deux fois dans un ordre séquentiel, et la concentration par chauffage à une température réduite et une pression réduite de 40 - 80°C et 100 - 350 mbar au moins une fois.

7. Méthode de préparation d'un extrait de pulpe de grenade selon la revendication 6, ladite méthode comprenant en outre un filtrage dans au moins une étape parmi celle postérieure à S1 et antérieure à S2, postérieure à S3 et antérieure à S4, et postérieure à S4.

8. Méthode de préparation d'un extrait de pulpe de grenade selon la revendication 6, dans laquelle l'extrait de pulpe de grenade améliore la tolérance chez un individu qui est sensible aux substances polyphénolées.

9. Méthode de préparation d'un extrait de pulpe de grenade selon la revendication 6, dans laquelle l'étape S4 comprend la concentration par chauffage sous 400 - 550 mbar à 70 - 85°C, la concentration par chauffage sous 550 - 750 mbar à 85 - 92°C, la concentration par chauffage sous 750 - 850 mbar à 92 - 100°C, la concentration par chauffage sous 250 - 350 mbar à 60 - 80°C, et la concentration par chauffage sous 100 - 250 mbar à 40 - 60°C.

10. Aliment fonctionnel diététique destiné à une utilisation permettant de soulager les symptômes de la ménopause chez la femme, comprenant l'extrait de pulpe de grenade préparé par la méthode de préparation selon la revendication 6 à titre d'ingrédient actif, dans lequel une dose quotidienne totale maximale de l'extrait de pulpe de grenade est inférieure ou égale à 15 ml.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101272602

- US 2013028994 A1

**Non-patent literature cited in the description**

- **KALIN MF ; ZUMOFF B.** *Steroids,* 1990, vol. 55, 330-352
- **TAMINI RT et al.** *Arch intern med,* 2006, vol. 166, 1483-1489
- **GERTIG DM.** *Menopause,* 2006, vol. 13, 178-184

- **STROM BL et al.** *Am J Epidemiol,* 2006, vol. 164, 775-586
- **ARAJR NL ; ATHANAZIO DA.** *Cad Saude Publica,* 2007, vol. 23, 2613-2622